# EUROPEAN PATENT APPLICATION

(11) **EP 4 413 936 A1**
(43) Date of publication of application: **14.08.2024**
(21) Application number: 24153113.6
(22) Date of filing: 22.01.2024
(51) Int. Cl.: A61B 18/12, A61B 18/14, A61B 18/00

(54) **SYSTEMS AND METHODS FOR ABLATION OF A THYROID NODULE**

(30) Priority: 09.02.2023 US 202363444270 P; 08.01.2024 US 202418406612
(71) Applicant: Medtronic Xomed, LLC, Jacksonville, FL 32216 (US)
(72) Inventor: PERGOLA, Nicholas F., Lafayette, 80026 (US); HISSONG, James Britton, Jacksonville, 32216 (US)
(74) Representative: Viering, Jentschura & Partner mbB Patent- und Rechtsanwälte

(57) **Abstract**

A system for ablating a thyroid nodule using a moving shot technique includes an ablation device configured to be inserted into a thyroid nodule. The ablation device has an electrode configured to be retracted along an axis defined through the thyroid nodule while delivering energy to the thyroid nodule. The system also includes an electrosurgical generator configured to delivery energy to the ablation device. An audio device is in communication with the electrosurgical generator. The audio device is configured to broadcast a sound correlated with at least one sensed tissue property such that the audio device changes the sound in response to a change in the at least one sensed tissue property.

## Description

### FIELD

The disclosure relates to tissue ablation. More specifically, the disclosure relates to ablation of a thyroid nodule using a "moving shot" technique.

### BACKGROUND

Conventional approaches to treating benign thyroid nodules and malignant thyroid cancers include removal of these tissues under general anesthesia by making an incision in the neck and resecting the tissues. However, aside from the inherent risks of undergoing general anesthesia, the patient also risks the possibility of undesirable side effects such as a residual neck scar, nerve damage, hypothyroid function, etc.

Improved approaches to reducing benign thyroid nodules and some malignant cancerous tumors involve percutaneously inserting, under the guidance of ultrasound imaging, a radio frequency (RF) ablation device under local anesthesia and ablating target tissue. Generally, tissue ablation involves inserting an ablation electrode (e.g., RF, microwave, or laser fiber) into target tissue and monitoring energy delivered into the target tissue to achieve a desired tissue temperature for a given period of time when using a traditional static ablation technique, or by producing a vigorous ablation environment using higher energy levels and moving the electrode through the tissue while ablating, which is known as a moving-shot technique. Using an ultrasound system including a suitable display, ablation of the target tissue can be observed such that the nodule is visible and the electrode can be observed percutaneously entering the surgical site and ablating the target tissue as the visualized target tissue changes in appearance due to heating of the target tissue caused by delivery of ablation energy to the target tissue. Using a traditional static ablation technique, the electrode is held in place to achieve a desired ablation zone and then can be moved to a new position and the process repeated until therapy for a target region is complete. In contrast to the traditional static ablation technique, the moving-shot technique involves delivering an increased energy level to tissue (e.g., increased relative to an energy level used in a traditional static ablation technique) to produce vigorous ablation and the electrode is moved either continuously through tissue or moved using a start-stop technique where the electrode is briefly held in place (e.g., held in place for a shorter amount of time relative to the above-noted traditional static ablation technique), moved to a new position, and the process repeated until therapy for a target region is complete. In some instances, this process is moderated by a specific parameter such as, for example, tissue temperature, which is controlled by energy delivery to the target tissue and feedback from the ablation device to the generator.

### SUMMARY

Provided in accordance with aspects of the present disclosure is a system for ablating a thyroid nodule. The system includes an ablation device configured to be inserted into a thyroid nodule. The ablation device has an electrode that is configured to be placed within the thyroid nodule and retracted along an axis defined through the thyroid nodule while delivering energy to the thyroid nodule. The system also includes an electrosurgical generator configured to delivery energy to the ablation device and to sense impedance of tissue being ablated by the ablation device. An audio device is in communication with the impedance sensor and is configured to broadcast a sound correlated with the tissue impedance sensed by the electrosurgical generator such that the audio device changes at least one of a volume, an intensity, a frequency, a periodicity, or a tone of the sound in response to a change in the impedance of the tissue sensed by the electrosurgical generator.

In an aspect of the present disclosure, the audio device is integral with the electrosurgical generator.

In another aspect of the present disclosure, the audio device is configured to broadcast a verbal indication associated with the tissue impedance sensed by the electrosurgical generator.

In another aspect of the present disclosure, the ablation device is a monopolar ablation device.

In another aspect of the present disclosure, the system also includes a return electrode electrically coupled to the electrosurgical generator and configured to be adhered to a patient for returning the energy from the thyroid nodule to the electrosurgical generator.

In still another aspect of the present disclosure, the system also includes an ultrasound imaging device configured to visualize the thyroid nodule and movement of the electrode within the thyroid nodule during ablation of the thyroid nodule.

In another aspect of the present disclosure, the audio device is configured to continuously broadcast the sound during delivery of the energy to the thyroid nodule.

Also provided in accordance with the present disclosure is a method for ablating a thyroid nodule. The method includes inserting an electrode of an ablation device into a first portion of a thyroid nodule along an axis defined through the thyroid nodule. The method also includes delivering electrosurgical energy from the electrode to the thyroid nodule while retracting the electrode along the axis toward a second portion of the thyroid nodule. The method also includes sensing at least one property of tissue being ablated by the electrode, broadcasting a sound correlated to the at least one property of the tissue, and controlling at least one of a volume, an intensity, a frequency, a periodicity, or a tone of the sound based on the at least one property of the tissue. The method also includes repeating the previous steps for at least one additional axis defined through the thyroid nodule until the ablation procedure is complete.

In an aspect of the present disclosure, the method also includes controlling a pace of retraction of the electrode based on at least one of the volume, the intensity, the frequency, the periodicity, or the tone of the sound.

In another aspect of the present disclosure, the method also includes controlling the pace of the retraction based on the sound, an ultrasound visualization of the electrode and the thyroid nodule, and a tactile feedback via a handle of the ablation device.

In still another aspect of the present disclosure, the method also includes broadcasting a verbal indication of a value of the at least one property of the tissue.

In yet another aspect of the present disclosure, retracting the electrode along the axis includes continuously retracting the electrode toward a superficial portion of the thyroid nodule.

In another aspect of the present disclosure, the method also includes imaging the electrode and the thyroid nodule during the ablation procedure using an ultrasound imaging device.

In still yet another aspect of the present disclosure, the method also includes interrupting delivery of the electrosurgical energy from the electrode to the thyroid nodule during insertion of the electrode into the first portion of the thyroid nodule.

In another aspect of the present disclosure, the method also includes changing at least one of the volume, the intensity, the frequency, the periodicity, or the tone of the sound in response to a change in the at least one property of the tissue.

In still another aspect of the present disclosure, the method also includes increasing at least one of the volume, the intensity, the frequency, the periodicity, or the tone of the sound in response to an increase of the at least one property of the tissue.

Another system for ablating a thyroid nodule using a moving shot technique is provided in accordance with the present disclosure and includes an ablation device configured to be inserted to a position adjacent tissue to be ablated. The ablation device has an electrode configured to be placed at a deepest portion of the tissue to be ablated and retracted along an axis defined through the tissue to be ablated for delivering energy to the tissue to be ablated. A sensor is disposed on the monopolar ablation device and is configured to sense at least one property of tissue being ablated by the ablation device. The system also includes an electrosurgical generator configured to delivery energy to the ablation device and an audio device operably coupled to the sensor. The audio device is configured to broadcast a sound correlated with the at least one tissue property sensed by the sensor such that the audio device changes at least one of a volume, an intensity, a frequency, or a tone of the sound in response to a change in the at least one property of the tissue sensed by the sensor.

In an aspect of the present disclosure, the audio device is configured to broadcast a verbal indication associated with the at least one property of the tissue sensed by the electrosurgical generator.

In another aspect of the present disclosure, the property of the sound is at least one of volume, intensity, frequency, periodicity, or tone.

In still another aspect of the present disclosure, the tissue to be ablated is a thyroid nodule and the electrode is configured to be retracted along an axis defined through the thyroid nodule while delivering energy to the thyroid nodule.

### BRIEF DESCRIPTION OF THE DRAWINGS

Aspects of the disclosure are described herein with reference to the accompanying drawings, wherein:
FIG. 1 is a schematic illustration of an ablation system including an ablation device, in accordance with aspects of the present disclosure;
FIG. 2 is a top plan view of a thyroid gland including depictions of conceptual ablation units and illustrating the ablation device of FIG. 1 inserted into a thyroid nodule for performing an ablation procedure, in accordance with aspects of the present disclosure;
FIG. 3 is a flowchart illustrating an example approach for performing an ablation procedure on a thyroid nodule using a "moving shot" technique, in accordance with aspects of the present disclosure; and
FIG. 4 is a schematic illustration of an exemplary robotic surgical system configured for use with the ablation system of FIG. 1, in accordance with aspects of the present disclosure.

### DETAILED DESCRIPTION

Embodiments of the disclosure are now described in detail with reference to the drawings in which like reference numerals designate identical or corresponding elements in each of the drawings. The aspects may be combined in any manner consistent with the functionality of the apparatus and/or method disclosed herein. As used herein, the term "clinician" refers to a doctor, a clinician, a nurse, or any other care provider and may include support personnel. Throughout this description, the term "proximal" will refer to the portion of the device or component thereof that is closer to the clinician and the term "distal" will refer to the portion of the device or component thereof that is farther from the clinician. As used herein, the term "exemplary" does not necessarily mean "preferred" and may simply refer to an example unless the context clearly indicates otherwise.

Terms including "generally," "about," "substantially," and the like, as utilized herein, are meant to encompass variations, e.g., manufacturing tolerances, material tolerances, use and environmental tolerances, measurement variations, design variations, and/or other variations, up to and including plus or minus 10 percent. Additionally, in the drawings and in the description that follows, terms such as front, rear, upper, lower, top, bottom, and similar directional terms are used simply for convenience of description and are not intended to limit the disclosure. In the following description, well-known functions or constructions are not described in detail to avoid obscuring the disclosure in unnecessary detail.

The present disclosure relates to ablation of a thyroid nodule using a "moving shot" technique. Ablation treatment of thyroid nodules and tumors typically includes inserting an ablation device at the midline of the neck in a lateral trajectory (e.g., using a trans-isthmic approach) for treatment of a nodule in either the right or left thyroid gland using a suitable form of ablation energy such as, for example, (RF), microwave, laser, cryogenics, or the like. Using ultrasound guidance, the insertion and placement of an electrode of the ablation device into the lateral aspect of the thyroid nodule can be visualized. Typically, the electrode is positioned at the distal tip of the ablation device. Once the electrode is in position, a high level of energy is applied and tissue ablation occurs, which can be visualized by the clinician via the ultrasound system. Additionally, as tissue boils in response to heating, steam is generated which may result in audible "tissue popping" caused by the formation, expansion, and rupturing of bubbles within the heated tissue. The resulting "tissue popping" manifests as vibration of the ablation device, which is felt by the clinician that is holding the handle of the ablation device to indicate to the clinician that the tissue and/or electrode temperature is high and ablation is occurring at a high level. The resulting "popping" sounds may be loud enough to be heard by the clinician and the patient. For a conscious patient, hearing their own tissue "popping" as a result of bubbles expanding and exploding during an ablation procedure may be disconcerting to the patient. Typically, the clinician will attempt to minimize "tissue popping" by retracting the ablation device along the same path in which it was inserted while applying energy via the electrode, thereby creating a path of ablated tissue within the thyroid nodule. This approach is typically referred to as a "moving shot" technique. In conventional ablation techniques used for organs such as the liver, the electrode tip is fixed to the center of the target tumor, which is ablated by conducted heat. However, the thyroid gland is surrounded by several critical structures such as, for example, the esophagus, the trachea, the recurrent laryngeal nerve, the carotid artery, the vagus nerve, and the cervical sympathetic ganglion. Using the fixed technique, the nodule periphery and immediate surrounding tissue may be undertreated or overtreated, depending on the treatment time and energy applied. To avoid these shortcomings, a thyroid nodule may be treated using the "moving shot" technique by dividing the nodule into multiple conceptual ablation units, which can be treated individually using a high energy level. Since applying a high energy level ablates more tissue in less time, the clinician can control the pace of movement of the ablation device for a given electrode size to create a volume of ablated tissue relatively quickly while minimizing "tissue popping".

Aside from the undesirable patient response to hearing "tissue popping" during an ablation procedure, the "tissue popping" may also be an indication of a loss of efficiency of energy delivery to target tissue since desiccation results in an increase in tissue impedance equaling a loss of energy delivery unless the generator accounts for that loss by delivering more power. Additionally, the "tissue popping" may create a void around the electrode as the tissue expands from the rupture caused by the steam, thereby resulting in a loss of tissue contact with the electrode and loss of energy delivery to the tissue.

To improve on the aforementioned shortcomings, the present disclosure is directed to an ablation system that provides audible feedback during performance of an ablation procedure using the "moving shot" technique. The audible feedback is correlated with a sensed tissue property (e.g., impedance, temperature, etc.) of the tissue being ablated at or near the electrode surface. For example, the audible feedback may be an intermittent or continuous audible sound (e.g., a beep, a tone, etc.) that increases in intensity, volume, frequency, periodicity, and/or tone as the sensed tissue property increases. Alternatively or additionally, the audible feedback may be a verbal indication (e.g., recorded or computer generated) providing information to the clinician about the sensed tissue property. For example, the verbal indication may be an impedance value. As tissue impedance increases, the efficiency of energy delivery to that tissue decreases. In this instance, the audible feedback may serve to alert the clinician to move the electrode and/or quicken the pace of retraction of the electrode to minimize inefficient energy delivery and/or "tissue popping", which will also result in shorter procedure times. The audible feedback may be provided via a suitable audio device (e.g., a speaker) that is either standalone or integral with the generator. The visual confirmation of ablation lesion generation in the ultrasound imaging, the audible feedback provided by the audio device, and the tactile feedback felt by the clinician in the handle of the RF ablation device enables the clinician to retract the electrode from the thyroid nodule at a pace that minimizes "tissue popping" while providing efficient energy delivery. This triangulation of feedback sources allows the clinician to perform an ablation procedure using the "moving shot" technique with overall shorter procedure times with the added potential for improved ablation volume reduction.

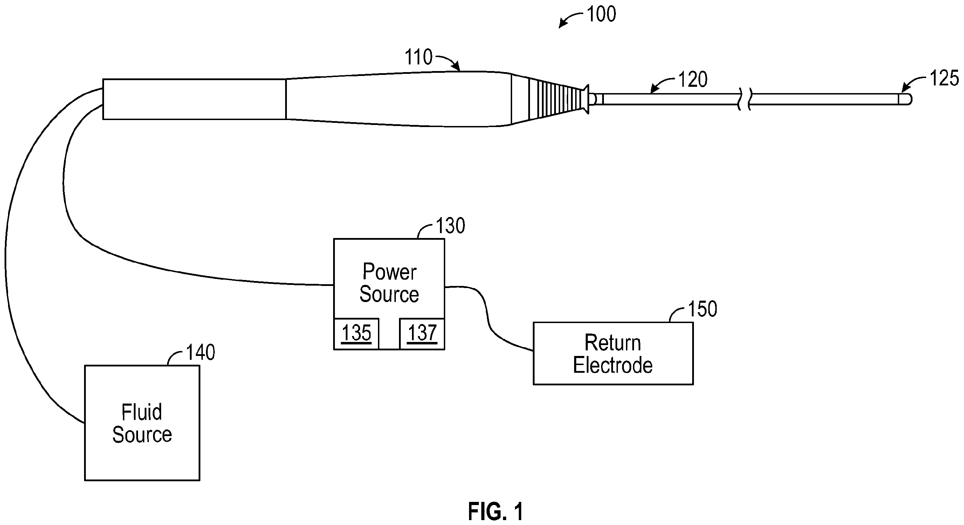

With reference to FIG. 1, an ablation system 10 according to aspects of the present disclosure is shown. The ablation system 10 generally includes an ablation device 100, an electrosurgical power source, e.g., generator 130, and a fluid source 140. In aspects of the present disclosure, the ablation device 100 may be a monopolar ablation device utilizing an electrode 125 disposed on a distal portion of an elongated shaft 120 of the ablation device 100 to contact and ablate tissue. In this scenario, the ablation system 10 may include a remote return electrode 150 (e.g., return pad) coupled to the generator 130, as shown in FIG. 1, and configured to be adhered to a patient. In other aspects of the present disclosure, the ablation device 100 may be a bipolar ablation device utilizing two electrodes (not shown) disposed on the ablation device 100 to deliver energy to tissue disposed between the two electrodes. In this scenario, the two electrodes are positioned at a distal portion of the elongated shaft 120 of the ablation device 100 axially spaced (e.g., 5-10 mm) from one another and electrically isolated from one another. For example, an insulative material (not shown) may be disposed on the elongated shaft 120 between the two electrodes to electrically insulate the two electrodes from each other.

The fluid source 140 is configured to supply coolant fluid (e.g., via fluid tubing) to the ablation device 100 to cool the elongated shaft 120 and/or the electrode 125 during an ablation procedure. In the scenario where the ablation device 100 is configured as a monopolar ablation device, the return electrode 150 is electrically coupled to the generator 130 and, during an ablation procedure, the return electrode 150 is adhered to the skin of a patient for returning, to the generator 130, electrosurgical energy that is delivered to the patient via the ablation device 100. In the scenario where the ablation device 100 is configured as a bipolar device, one of the two electrodes serves the same purpose as the return electrode 150 of the above-noted monopolar configuration by returning, to the generator 130, energy delivered to tissue by the other of the two electrodes, thereby obviating the need for a remote return electrode adhered to the patient.

Commercially available ablation systems for use with the present disclosure include, for example, the Cool-tip^{™} RF Ablation System and the Accurian^{™} RF Ablation Platform both available from Medtronic plc of Dublin, Ireland. Although aspects of the present disclosure are described in terms of using RF energy to ablate tissue, such description should not be considered limiting. It is contemplated that the ablation device 100 and the generator 130 of the present disclosure may be configured for use with other suitable forms of ablation energy such as, for example, microwave, laser, ultrasonic, and/or cryogenics.

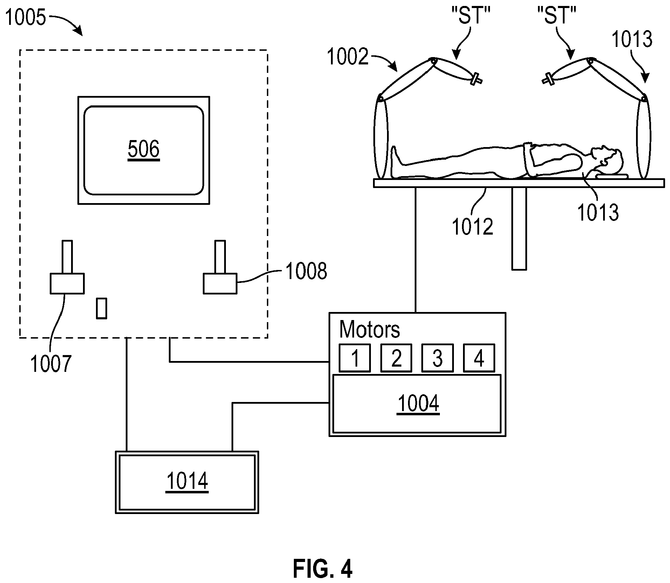

The ablation device 100 includes a handle 110 coupled to the elongated shaft 120. The handle 110 is configured to be gripped and manipulated by a clinician during an ablation procedure, although non-handle configurations are also contemplated, e.g., for mounting the ablation device 100 and/or attaching the ablation device 100 to a surgical robot arm (see FIG. 4). The electrode 125 is disposed at a distal end portion of the elongated shaft 120 and is configured for contacting and ablating tissue. The electrode 125 is configured to electrically couple to the generator 130 for providing electrosurgical energy (e.g., RF, microwave, laser, ultrasonic, cryogenic, etc.) to the electrode 125. The generator 130 may be configured to operate in one or both of a monopolar configuration or a bipolar configuration and include suitable outputs for delivering electrosurgical energy to the ablation device 100 and/or suitable inputs for returning electrosurgical energy to the generator 130. The generator 130 includes an impedance measurement circuit 137 configured to measure impedance of tissue being ablated by the electrode 125.

In some aspects of the present disclosure, the elongated shaft 120 of the ablation device 100 may include one or more sensors (not explicitly shown) configured to sense properties of surrounding tissue (e.g., tissue temperature, tissue impedance, etc.) and/or properties of the ablation device 100 (e.g., temperature of the elongated shaft 120 and/or the electrode 125). The one or more sensors may include, for example, a temperature-sensitive sensor (e.g., thermocouple, thermistor, etc.).

The generator 130 may include an audio device 135 that provides audible feedback correlated to one or more sensed properties (e.g., impedance, temperature, etc.) of the tissue being ablated. For example, during a "moving shot" ablation procedure, the clinician may be alerted to change or maintain a pace of movement (e.g., retraction) of the electrode 125 of the ablation device 100 in accordance with the sensed tissue property. The audio device 135 may be, for example, a speaker that broadcasts an intermittent or continuous sound (e.g., a beep, a tone, or the like) that changes in volume, intensity, frequency, periodicity, and/or tone as the sensed tissue property changes. In aspects of the present disclosure, the sound continuously or intermittently broadcast by the audio device 135 may be a verbal auditory signal that verbally indicates a numerical value of the sensed tissue property. For example, the audio device 135 may broadcast a recorded voice verbalizing a specific value of sensed tissue impedance value (e.g., "400 ohms"). With this feature in mind, the clinician may set upper and/or lower impedance limits such that the audio device 135 may verbally indicate the sensed tissue impedance value at the start of an ablation procedure, verbally indicate the sensed tissue impedance value when the upper limit is exceeded, and verbally indicate the sensed tissue impedance has fallen below the lower limit. Additionally or alternative, the audio device 135 may simply provide a suitable verbal indication (e.g., a sound) that the sensed tissue impedance value has exceeded the upper limit and/or that the sensed tissue impedance value has fallen below the lower limit.

In an aspect of the present disclosure, the impedance measurement circuit 137 and/or the one or more sensors of the elongated shaft 120 may be in electrical communication with the audio device 135 such that the sensed tissue property (e.g., impedance, temperature, etc.) and/or a sensed property of the ablation device 100 (e.g., temperature of the elongated shaft 120 and/or the electrode 125) is correlated to the sound broadcast by the audio device 135. In other aspects of the present disclosure, the audio device 135 may be located on the ablation device 100 or the fluid source 140. The audio device 135 may also be a standalone device separate from the ablation device 100, the generator 130, and the fluid source 140.

In another aspect of the present disclosure, the audio device 135 may be configured to broadcast a sound (e.g., a beep) upon the sensed tissue property meeting or exceeding a predetermined threshold value. Such a predetermined threshold may be stored in a memory of the generator 130. For example, if tissue impedance meets or exceeds a predetermined threshold impedance value, the audio device 135 will broadcast a sound that serves to alert the clinician to quicken the pace of movement of the electrode 125 and/or to move the electrode 125 from its current location since energy is no longer being delivered efficiently to the tissue. In this scenario, the audio device 135 may be silent prior to the sensed tissue property meeting or exceeding the predetermined threshold and only broadcast a sound upon the sensed tissue property meeting or exceeding the predetermined threshold. Alternatively, the audio device 135 may broadcast a sound prior to the sensed tissue property meeting or exceeding the predetermined threshold and change a volume, an intensity, a frequency, periodicity, and/or a tone of the sound upon the sensed tissue property meeting or exceeding the predetermined threshold. In aspects of the present disclosure, the predetermined threshold may be an impedance value that is calculated at the time of a procedure and based on a rise in impedance that is a specific percentage above the impedance at the start of the ablation procedure or at an early phase of the ablation procedure when tissue impedance level drops immediately following initiation of ablation energy.

In aspects of the present disclosure, the generator 130 may include one or more processors and one or more processor-readable media (e.g., memory) storing instructions. The instructions may be executed by the processor, which may include one or more digital signal processors (DSPs), general-purpose microprocessors, application-specific integrated circuits (ASICs), field programmable logic arrays (FPGAs), or other equivalent integrated or discrete logic circuitry. Accordingly, the term "processor" as used herein may refer to any of the foregoing structures or any other physical structure suitable for implementation of the described techniques. Also, the techniques could be fully implemented in one or more circuits or logic elements.

In one or more examples, the described techniques may be implemented in hardware, software, firmware, or any combination thereof. If implemented in software, the functions may be stored as one or more instructions or code on a computer-readable medium and executed by a hardware-based processing unit. Processor-readable media may include non-transitory processor-readable media, which corresponds to a tangible medium such as data storage media (e.g., RAM, ROM, EEPROM, flash memory, or any other medium that can be used to store desired program code in the form of instructions or data structures and that can be accessed by a processor).

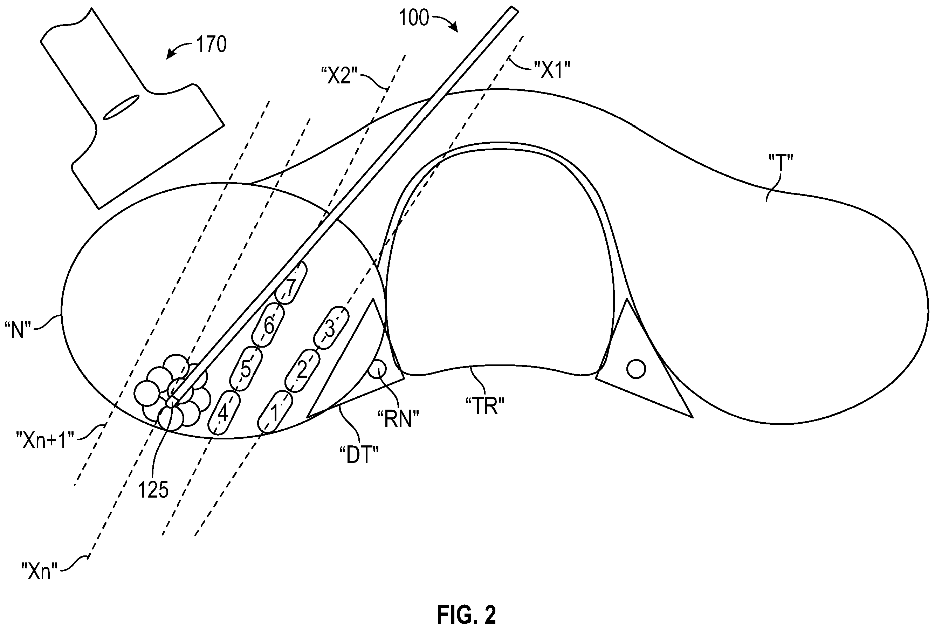

Referring now to FIG. 2, a depiction of a patient's thyroid gland "T" is shown in proximity to the patient's trachea "TR" and the "danger triangle" (referenced as "DT") including the recurrent laryngeal nerve "RN". The electrode 125 of the ablation device 100 is shown percutaneously inserted into a thyroid nodule "N" using the trans-isthmic approach under ultrasound guidance via an ultrasound imaging device 170. As would be understood, the ultrasound imaging device 170 is configured for coupling to a suitable display monitor (not shown) for visualizing, during an ablation procedure, the thyroid nodule "N" and the portion of the ablation device 100 percutaneously placed within the thyroid nodule "N". Multiple conceptual ablation units are shown within the thyroid nodule and numbered (e.g., 1-7) in order to illustrate a standard "moving shot" technique for ablating the thyroid nodule "N". The electrode 125 of the ablation device 100 is inserted through the isthmus such that the electrode 125 and the thyroid nodule "N" are visualized using the ultrasound imaging device 170. The electrode 125 is inserted into the thyroid nodule "N" and placed at the deepest portion of the thyroid nodule "N" at conceptual ablation unit "1" where RF energy is delivered to the thyroid nodule "N" via the electrode 125. While delivering energy to tissue, the clinician retracts the electrode 125 toward the most superficial portion of the thyroid nodule "N" in accordance with the order of numbering of each conceptual ablation unit. For example, the clinician will retract the electrode 125 along a first axis "X1" from conceptual ablation unit "1" to conceptual ablation unit "2" and subsequently to conceptual ablation unit "3" to ablate the thyroid nodule "N" unit-by-unit along the first axis "X1". Following ablation of conceptual ablation unit "3", the clinician will move the electrode 125 along a second axis "X2" adjacent to the first axis "X1" to once again place the electrode at the deepest portion of the thyroid nodule "N" at conceptual ablation unit "4", which is adjacent to conceptual ablation unit "1" at the deepest portion of the thyroid nodule "N". In aspects of the present disclosure, energy delivery from the generator 130 to the electrode 125 may be interrupted during "forward" movement of the electrode 125 to place the electrode 125 at the deepest portion of the thyroid nodule "N". For example, once ablation of the thyroid nodule "N" along the first axis "X1" is complete, energy delivery to the electrode 125 may be temporarily interrupted following ablation of conceptual ablation unit "3" and during insertion (e.g., forward motion along the second axis "X2") of the electrode 125 to place the electrode 125 at the deepest portion of the thyroid nodule "N" at conceptual ablation unit "4". Once the electrode 125 is placed at conceptual ablation unit "4", RF energy is again delivered to the electrode 125 and the electrode 125 is retracted along the second axis "X2" unit-by-unit in accordance with the order of numbering of each conceptual ablation unit (e.g., from conceptual ablation unit "4" to conceptual ablatio unit "7") until ablation of the thyroid nodule "N" along the second axis "X2" is complete. This "moving shot" technique is performed for subsequent adjacent axes (e.g., axis "Xn", axis "Xn+1" and so on) until the thyroid nodule "N" is ablated, unit-by-unit and axis-by-axis, to completion. It is contemplated that the audio device 135 may continue to broadcast a sound during interruption of energy delivery to the electrode 125 so that the disclosed audible feedback feature will continue to operate when the clinician interrupts application of energy to tissue, moves the electrode 125, and reinitiates application of energy to tissue.

According to an aspect of the present disclosure, the audio device 135 provides audible feedback correlated to an impedance of the tissue being ablated during an ablation procedure performed using the "moving shot" technique. For example, the audio device 135 broadcasts a sound that will change in accordance with the impedance of the tissue being ablated as sensed by the impedance measurement circuit 137 of the generator 130. As the sensed impedance increases, for example, the sound broadcast by the audio device 135 may increase in volume, intensity, frequency, periodicity, and/or tone. If the clinician is moving the electrode 125 too slowly such that impedance is increasing to a level that prevents efficient energy delivery, the sound broadcast by the audio device 135 may serve as a prompt to the clinician to quicken the pace of movement of the electrode 135. Likewise, if the clinician is moving the electrode 125 too quickly, the sound broadcast by the audio device 135 may serve as a prompt to the clinician to slow the pace of movement of the electrode 135 to ensure a complete ablation volume at one or more conceptual ablation units. In this way, the clinician can utilize the sound broadcast by the audio device 135 to control the pace of retraction of the electrode 125 during a "moving shot" ablation procedure.

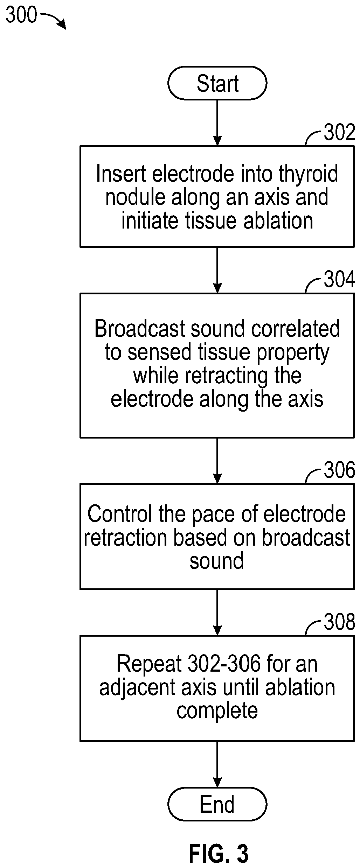

Turning now to FIG. 3, an example approach 300 is shown for performing an ablation procedure using a "moving shot" technique to ablate a thyroid nodule audible feedback as a guide for controlling the pace of retraction of the electrode 125 while ablating the thyroid nodule "N". In aspects of the present disclosure, the clinician may control the pace of retraction of the electrode 125 based on other feedback either individually or in combination with the audible feedback provided by the audio device 135. For example, the clinician may triangulate the visual confirmation of ablation lesion generation in the ultrasound imaging, the audible feedback provided by the audio device, and the tactile feedback felt by the clinician in the handle of the RF ablation device 100 to enable retraction of the electrode 125 along a given axis at a pace that serves to minimize "tissue popping" while providing efficient energy delivery to the thyroid nodule "N". Although the example approach 300 is described in terms of ablating the thyroid nodule "N", it should be appreciated that this procedure is merely provided as an example and that approach 300 may also be applied to other ablation procedures (e.g., liver ablation, lung ablation, etc.) where using the "moving shot" technique may be practical.

At block 302, the elongated shaft 120 of the ablation device 100 is percutaneously inserted, under ultrasound guidance, into the thyroid nodule "N" using a trans-isthmic approach such that the electrode 125 of the ablation device 100 is inserted into the thyroid nodule "N" and placed at the deepest portion of the thyroid nodule "N". Once the electrode 125 is placed at the deepest portion of the thyroid nodule "N", electrosurgical energy (e.g., RF energy) is delivered from the generator 130 to the electrode 125 to initiate ablation of the thyroid nodule "N". As the electrode 125 is ablating tissue, one or more properties of the tissue being ablated are sensed. In an aspect of the present disclosure, the sensed property of the tissue being ablated is impedance sensed by the impedance measurement circuit 137 of the generator 130. In another aspect of the present disclosure, the sensed property of the tissue being ablated is temperature sensed by a temperature-sensitive sensor (e.g., thermocouple, thermistor, etc.) disposed on the elongated shaft 120 of the ablation device 100.

At block 304, the clinician retracts the electrode 125 from the deepest portion of the thyroid nodule along the first axis "X1" toward the most superficial portion of the thyroid nodule "N" while the audio device 135 intermittently or continuously broadcasts a sound that is correlated to the sensed tissue property such that the sound changes in response to a change in the sensed tissue property. At block 306, the clinician controls the pace of retraction of the electrode 125 in response to the sound broadcast by the audio device 135. For example, as sensed tissue impedance increases the sound will increase in one or more of volume, intensity, frequency, periodicity, or tone. Likewise, as sensed tissue impedance decreases the sound will decrease in one or more of volume, intensity, frequency, periodicity, or tone. If sensed tissue impedance is not changing, the sound will not change. The sound may initially be set to a default state corresponding to a moderate volume, intensity, frequency, periodicity, and/or tone. The sound will increase or decrease from the default state to serve as an alert to the clinician to change the pace of movement of the electrode 125 or the sound will remain at the default state to serve as an indication to the clinician to maintain the pace of movement of the electrode 125. As a practical example, the clinician may momentarily stop movement of the electrode 125 at any one or more of the conceptual ablation units to ensure ablation at that conceptual ablation unit is complete. In this scenario, as the sensed tissue impedance and/or temperature increases, the sound broadcast by the audio device 135 will increase in volume, intensity, frequency, periodicity, and/or tone, which serves as an alert for the clinician to continue retracting the electrode 125 along the axis toward the next conceptual ablation unit.

At block 308, once ablation of the thyroid nodule "N" along an axis (e.g., axis "X1") is complete, the clinician will repeat blocks 302-306 for an adjacent axis (e.g., axis "X2", "Xn", "Xn+1", etc.) until ablation of the thyroid nodule "N" is complete.

Turning now to FIG. 4, a robotic surgical system 1000 configured for use in accordance with the present disclosure is shown. Aspects and features of robotic surgical system 1000 not germane to the understanding of the present disclosure are omitted to avoid obscuring the aspects and features of the present disclosure in unnecessary detail.

Robotic surgical system 1000 generally includes a plurality of robot arms 1002, 1003; a control device 1004; and an operating console 1005 coupled with control device 1004. Operating console 1005 may include a display device 1006, which may be set up in particular to display three-dimensional images; and manual input devices 1007, 1008, by means of which a clinician, e.g., a clinician, may be able to telemanipulate robot arms 1002, 1003 in a first operating mode. Robotic surgical system 1000 may be configured for use on a patient 1013 lying on a patient table 1012 to be treated in a minimally invasive manner. Robotic surgical system 1000 may further include a database 1014, in particular coupled to control device 1004, in which are stored, for example, pre-operative data from patient 1013 and/or anatomical atlases.

Each of the robot arms 1002, 1003 may include a plurality of members, which are connected through joints, and a mounted device which may be, for example, a surgical tool "ST." The surgical tools "ST" may include, for example, the ablation device 100 of the present disclosure, thus providing any of the above-detailed functionality on a robotic surgical system 1000.

Robot arms 1002, 1003 may be driven by electric drives, e.g., motors, connected to control device 1004. The motors, for example, may be rotational drive motors configured to provide rotational inputs to accomplish a desired task or tasks. Control device 1004, e.g., a computer, may be configured to activate the motors, in particular by means of a computer program, in such a way that robot arms 1002, 1003, and, thus, their mounted surgical tools "ST" execute a desired movement and/or function according to a corresponding input from manual input devices 1007, 1008, respectively. Control device 1004 may also be configured in such a way that it regulates the movement of robot arms 1002, 1003 and/or of the motors.

Control device 1004, more specifically, may control one or more of the motors based on rotation, e.g., controlling to rotational position using a rotational position encoder (or Hall effect sensors or other suitable rotational position detectors) associated with the motor to determine a degree of rotation output from the motor and, thus, the degree of rotational input provided. Alternatively or additionally, control device 1004 may control one or more of the motors based on torque, current, or in any other suitable manner.

While several aspects of the disclosure have been shown in the drawings, it is not intended that the disclosure be limited thereto, as it is intended that the disclosure be as broad in scope as the art will allow and that the specification be read likewise. Therefore, the above description should not be construed as limiting, but merely as exemplifications of particular aspects. Those skilled in the art will envision other modifications within the scope and spirit of the claims appended hereto.

For example, further embodiments of the invention are as follows:
According to a 1st further embodiment, there is provided a method for ablating a thyroid nodule, the method comprising: a) inserting an electrode of an ablation device into a first portion of a thyroid nodule along an axis defined through the thyroid nodule; b) delivering electrosurgical energy from the electrode to the thyroid nodule while retracting the electrode along the axis toward a second portion of the thyroid nodule; c) sensing at least one property of tissue being ablated by the electrode; d) broadcasting a sound correlated to the at least one property of the tissue; e) controlling at least one of a volume, an intensity, a frequency, a periodicity, or a tone of the sound based on the at least one property of the tissue; and repeating the steps a) through e) for at least one additional axis defined through the thyroid nodule until the ablation procedure is complete.

According to a 2nd further embodiment, there is provided the method according to the 1st further embodiment, further comprising controlling a pace of retraction of the electrode based on at least one of the volume, the intensity, the frequency, the periodicity, or the tone of the sound.

According to a 3rd further embodiment, there is provided the method according to the 1st or 2nd further embodiment, further comprising controlling the pace of the retraction based on the sound, an ultrasound visualization of the electrode and the thyroid nodule, and a tactile feedback via a handle of the ablation device.

According to a 4th further embodiment, there is provided the method according to one of the 1st to 3rd further embodiments, further comprising broadcasting a verbal indication of a value of the at least one property of the tissue.

According to a 5th further embodiment, there is provided the method according to one of the 1st to 4th further embodiments, wherein retracting the electrode along the axis includes continuously retracting the electrode toward a superficial portion of the thyroid nodule.

According to a 6th further embodiment, there is provided the method according to one of the 1st to 5th further embodiments, further comprising imaging the electrode and the thyroid nodule during the ablation procedure using an ultrasound imaging device.

According to a 7th further embodiment, there is provided the method according to one of the 1st to 6th further embodiments, further comprising interrupting delivery of the electrosurgical energy from the electrode to the thyroid nodule during insertion of the electrode into the first portion of the thyroid nodule.

According to an 8th further embodiment, there is provided the method according to one of the 1st to 7th further embodiments, further comprising changing at least one of the volume, the intensity, the frequency, the periodicity, or the tone of the sound in response to a change in the at least one property of the tissue.

According to a 9th further embodiment, there is provided the method according to one of the 1st to 8th further embodiments, further comprising increasing at least one of the volume, the intensity, the frequency, the periodicity, or the tone of the sound in response to an increase of the at least one property of the tissue.

According to a 10th further embodiment, there is provided a system for ablating tissue, the system comprising: an ablation device configured to be inserted to a position adjacent tissue to be ablated, the ablation device having an electrode configured to be placed at a first portion of the tissue to be ablated and retracted along an axis defined through the tissue to be ablated for delivering energy to the tissue to be ablated; an electrosurgical generator configured to deliver energy to the ablation device and to sense at least one property of tissue being ablated by the ablation device ; and an audio device operably coupled to the electrosurgical generator and configured to broadcast a sound correlated with the at least one tissue property sensed by the electrosurgical generator such that the audio device changes a property of the sound in response to a change in the at least one property of the tissue sensed by the electrosurgical generator.

According to an 11th further embodiment, there is provided the system according to the 10th further embodiment, wherein the audio device is configured to broadcast a verbal indication associated with the at least one property of the tissue sensed by the electrosurgical generator.

According to a 12th further embodiment, there is provided the system according to the 10th or 11th further embodiment, wherein the property of the sound is at least one of volume, intensity, frequency, periodicity, or tone.

According to a 13th further embodiment, there is provided the system according to one of the 10th to 12th further embodiments, wherein the tissue to be ablated is a thyroid nodule and the electrode is configured to be retracted along an axis defined through the thyroid nodule while delivering energy to the thyroid nodule.

## Claims

1. A system for ablating a thyroid nodule, comprising:
an ablation device configured to be inserted into a thyroid nodule, the ablation device having an electrode configured to be placed within the thyroid nodule and retracted along an axis defined through the thyroid nodule while delivering energy to the thyroid nodule;
an electrosurgical generator configured to deliver electrosurgical energy to the ablation device and to sense impedance of tissue being ablated by the ablation device; and
an audio device in communication with the electrosurgical generator and configured to broadcast a sound correlated with the tissue impedance sensed by the electrosurgical generator such that the audio device changes at least one of a volume, an intensity, a frequency, a periodicity, or a tone of the sound in response to a change in the impedance of the tissue sensed by the electrosurgical generator.

2. The system according to claim 1, wherein the audio device is integral with the electrosurgical generator.

3. The system according to claim 1 or 2, wherein the audio device is configured to broadcast a verbal indication associated with the tissue impedance sensed by the electrosurgical generator.

4. The system according to one of claims 1-3, wherein the ablation device is a monopolar ablation device.

5. The system according to claim 4, further comprising a return electrode electrically coupled to the electrosurgical generator and configured to be adhered to a patient for returning the energy from the thyroid nodule to the electrosurgical generator.

6. The system according to one of claims 1-5, further comprising an ultrasound imaging device configured to visualize the thyroid nodule and movement of the electrode within the thyroid nodule during ablation of the thyroid nodule.

7. The system according to one of claims 1-6, wherein the audio device is configured to continuously broadcast the sound during delivery of the energy to the thyroid nodule.
